# EUROPEAN PATENT APPLICATION

(11) **EP 4 806 547 A1**
(43) Date of publication of application: **16.09.2026**
(21) Application number: 26164686.3
(22) Date of filing: 24.03.2021
(51) Int. Cl.: B01J 3/00, A23B 2/00, A61L 2/26

(54) **CONTAINER AND LOAD BASKET FOR THERMAL MANAGEMENT FOR PROCESSING IN HIGH PRESSURE APPLICATION**

(30) Priority: 27.03.2020 US 202063001047 P
(62) Document number(s) of the earlier application(s) in accordance with Art. 76 EPC: 21724399.7 / 4 171 790
(71) Applicant: JBT Marel Corporation, Chicago, IL 60602 (US)
(72) Inventor: Pelfrey, Rick D., Middletown, 45044 (US); Pereira, Ryan, Middletown, 45044 (US); Zhang, Limin, Middletown, 45044 (US); Malmberg, Karl Magnus, Middletown, 45044 (US); Viberg, Jan Olof, Middletown, 45044 (US)
(74) Representative: AWA Sweden AB

(57) **Abstract**

A generally cylindrically shaped container (70; 80; 90; 100) for receiving and supporting products for high pressure processing (HPP) with a pressurized liquid processing medium within a HPP chamber is provided.

## Description

### CROSS-REFERENCE TO RELATED APPLICATION

This patent application claims priority of U.S. Patent Application Serial No. 63/001,047, filed on March 27, 2020, the entire disclosure of which is hereby incorporated by reference herein for all purposes.

### BACKGROUND

High pressure processing (also "HPP") is used to reduce the microbial load on foods, beverages, cosmetics, pharmaceuticals and other products without significantly altering the characteristics of the processed product. The pressure level required for HPP to be successful is typically at least 4,000 bar.

Traditional equipment for treatment of beverages and other liquids as well as pumpable foods and other products by HPP is based on the processing of the products after having been placed as individual units into flexible packaging, for example, bottles, cartons, or pouches. The individual units are grouped or consolidated within a larger reusable load basket which is sized and shaped to fit into a wire wound high pressure vessel (also referred to as "wire wound vessel" or "high pressure vessel").

Such high pressure vessel is filled with water which serves as the pressurizing medium. Once the wire wound vessel has been filled and closed, high capacity pumps introduce additional water into the pressure vessel so that the pressure therein is increased from about 4,000 to 10,000 bar. This pressure is maintained for a sufficient length of time, from a few seconds to several minutes, to reduce the microbial load on the products being treated. The particular pressure level and the time duration of such pressure are specific to the product being processed.

Once the desired level of inactivation of the microorganisms has been achieved, the pressure in the vessel is released and the load basket is removed from therein so that the individual packages can be extracted. The processed product has, after being exposed to high pressure and hold time, been pasteurized, the microbial load has been reduced, and an extended shelf life has been achieved.

HPP has also been used in the "bulk" processing of the product, especially pumpable products. The pumpable products are placed in a large flexible bag or bladder type container located inside the pressure vessel thereby to occupy a significant proportion of the useful volume within the pressure vessel. The bladder is then pressurized by the pressure media in much the same way as if a load basket were used.

During an HPP cycle, the processing media, as well as the product being processed, are subject to an adiabatic temperature rise by virtue of the pressure being applied thereto. This increase in temperature is typically 3°C per 1,000 bar. Thus, at an operating pressure of 6,000 bar, the temperature increase can be approximately 18°C.

Different materials exhibit different adiabatic properties so that with increase in pressure, the temperature increase may vary. This can create a layered or stratified temperature distribution along the height or depth of the pressure media or product processed in an HPP pressure vessel. This thermal distribution in the pressure vessel can result in a significant temperature variation range in the product being processed. Applicant has identified layering effects that under certain circumstances may have a negative effect on the end result of the final product being processed in an HPP pressure vessel. The lower portion of the contained pressure media and product may be significantly colder than the upper portion of the contained pressure media and product. In some tests conducted by applicant, the upper 60% of the pressurized volume was more temperature homogenized than the lower 40% of the pressurized volume.

Applicant's tests also have indicated that the stratification or layering of the temperature of the processing media or pumpable product during HPP is due to changes in the density of the processing medium or pumpable product caused by the pressure applied therein. As a result, the colder processing media or pumpable product tends to sink while the warmer processing media or pumpable product tends to rise.

The foregoing temperature stratification or layering may not be of significant concern or an important factor in a normal or standard HPP process. However, if HPP is carried out at a specific temperature, especially within a narrow temperature range, for example, when processing dairy products to achieve a desired pasteurization level, then the temperature stratification of the processing medium as well as the dairy product can be of vital significance. The present disclosure seeks to address this stratification of the temperature of the processing medium and/or product being processed in an HPP system.

DE 20 2020 101053 U1 discloses a structure of a high temperature cooking device.

CA 2 221 680 A1 discloses a microwave waste sterilizer and a method of its use.

US 2018/168201 A1 discloses a load basket with a removable end cap.

CA 3 109 877 A1 discloses a system for protecting and securing bags for bulk high pressure processing equipment and an associated method.

### DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 discloses a load basket for receiving and holding packaged products for HPP;
FIGURE 2 is a pictorial view of an embodiment of an HPP load basket in accordance with the present disclosure;
FIGURE 3 is a cross-sectional view of FIGURE 2, taken along lines 3-3 thereof;
FIGURE 4 is a cross-sectional view similar to FIGURE 3 of a further embodiment of an HPP load basket in accordance with the present disclosure;
FIGURE 5 is a cross-sectional view of a further embodiment of a further HPP load basket in accordance with the present disclosure;
FIGURE 6 is a cross-sectional view similar to FIGURE 5 of a further embodiment of the present disclosure;
FIGURE 7 is an isometric view of an HPP load basket in accordance with the present disclosure;
FIGURE 8 is a cross-sectional view of FIGURE 7 taken along lines 8-8 thereof and shown within a pressure vessel, also in cross-section;
FIGURE 9 is a longitudinal cross-sectional view of the HPP basket of FIGURE 7 taken along lines 9-9 thereof;
FIGURE 10 is an embodiment of a bulk container in accordance with the present disclosure;
FIGURE 11 is a cross-sectional view of FIGURE 10 taken along lines 12-12 thereof;
FIGURE 12 is a view similar to FIGURE 10, but in accordance with a further embodiment to the present disclosure;
FIGURE 13 is a cross-sectional view of FIGURE 12 taken along lines 14-14 thereof;
FIGURE 14 is a pictorial view of a further HPP bulk container in accordance with the present disclosure;
FIGURE 15 is an exploded view of FIGURE 14; and
FIGURES 16, 17, and 18 are similar to FIGURES 7, 8, and 9, but with the addition of bulk container or bladder positioned therein to occupy the interior of the load basket.

### DETAILED DESCRIPTION

In the following description and in the accompanying drawings, corresponding systems, assemblies, apparatus and units may be identified by the same part number, but with an alpha suffix. The descriptions of the parts/components of such systems assemblies, apparatus, and units that are the same or similar are not repeated so as to avoid redundancy in the present application.

The description set forth below in connection with the appended drawings, where like numerals reference like elements, is intended as a description of various embodiments of the disclosed subject matter and is not intended to represent the only embodiments. Each embodiment described in this disclosure is provided merely as an example or illustration and should not be construed as preferred or advantageous over other embodiments. The illustrative examples provided herein are not intended to be exhaustive or to limit the disclosure to the precise forms disclosed. Similarly, any steps described herein may be interchangeable with other steps, or combinations of steps, in order to achieve the same or substantially similar result.

In the following description, numerous specific details are set forth in order to provide a thorough understanding of exemplary embodiments of the present disclosure. It will be apparent to one skilled in the art, however, that many embodiments of the present disclosure may be practiced without some or all of the specific details. In some instances, well known process steps have not been described in detail in order not to unnecessarily obscure various aspects of the present disclosure. Further, it will be appreciated that embodiments of the present disclosure may employ any combination of features described herein.

The present application may include references to "directions," such as "forward," "rearward," "front," "back," "ahead," "behind," "upward," "downward," "above," "below," "horizontal," "vertical," "top," "bottom," "right hand," "left hand," "in," "out," "extended," "advanced," "retracted," "proximal," and "distal." These references and other similar references in the present application are only to assist in helping describe and understand the present disclosure and are not intended to limit the present invention to these directions.

The present application may include modifiers such as the words "generally," "approximately," "about," or "substantially." These terms are meant to serve as modifiers to indicate that the "dimension," "shape," "temperature," "time," or other physical parameter in question need not be exact, but may vary as long as the function that is required to be performed can be carried out. For example, in the phrase "generally circular in shape," the shape need not be exactly circular as long as the required function of the structure in question can be carried out.

The present application refers to "processing medium" used in the HPP system for applying high pressure to the product being processed. Such processing medium is also referred to in the application as processing fluid or processing water as well as referred to as pressurized/pressure medium, pressurized/pressure fluid or pressurized water. All of these terms are to be used interchangeably.

In addition, the present application refers to the pressure vessel of an HPP apparatus. Such pressure vessel is also referred to as wire wound vessel or simply vessel. These terms are to be considered as synonymous.

Also, in the present application the term "container" shall generically refer to both open type load baskets and closed containers. In this specification the term "load basket" will be used to refer to an open type container and the term "container" will be used to refer to a closed container, so as to help avoid confusion between these types of containers.

Further, the present application refers to a "product" or "products" that are subjected to or treated by HPP using the containers of the present disclosure. Such product(s) may include all manner of foods, including pumpable foods or beverages, as well as non-food products, such as cosmetics, pharmaceuticals, and organic materials and substances, wherein the control of pathogens is desirable.

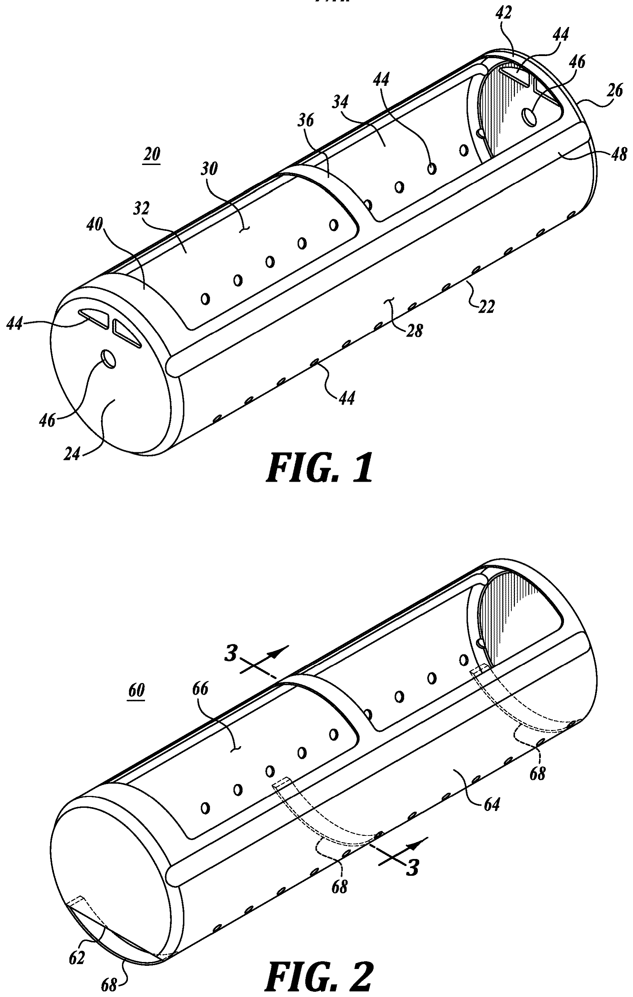

As shown in FIGURE 1, a load basket 20 for high pressure processing includes a generally cylindrical body 22 having first and second ends 24, 26 and a curvilinear sidewall 28 extending therebetween. The cylindrical body 22 may be constructed of any material suitable for high pressure processing, whether a metal or a polymer. While the body 22 is illustrated with a generally cylindrical shape with a generally circular cross-section, the body 22 could have different cross-sectional forms such as square, rectangular, triangular, hexagonal or any other suitable polygonal shape. As shown, the cylindrical body 22 defines an interior volume 30 for receiving packaged food products or other products to be processed in a high-pressure processing chamber (not shown). Volume 30 could also be filled with a thin walled liner bag for bulk processing.

With continued reference to FIGURE 1, the load basket 20 may include first and second top openings 32, 34 in the sidewall 28 of the cylindrical body 22, through which the packaged food products or other products may be inserted into and removed from the basket 20. As shown, the first and second top openings 32, 34 are separated by a middle bridging portion 36 of the sidewall 28, which may be used to provide a gripping point for lifting the load basket 20 or to add rigidity to the cylindrical shape of the load basket. The first and second top openings 32, 34 may terminate at or near the first and second ends 24, 26, respectively.

As shown in FIGURE 1, first and second outer bridging portions 40, 42 of the sidewall 28 are provided adjacent the first and second top openings 32, 34, respectively, to assist in maintaining the generally cylindrical shape of the cylindrical body 22, and/or to also provide stiffness or rigidity to the cylindrical body 22. As shown, the widths of the first and second outer bridging portions 40, 42 may be different from each other. The bridging portions 40, 42 also may be used as a gripping point(s) for lifting or otherwise manipulating the cylindrical body 22.

A plurality of fluid passage holes 44 may be provided through the sidewall 28 of the cylindrical body 22 to allow the pressure-transmitting medium of the high pressure processing chamber to fill the interior 30 with pressure media before the pressure increase initiates. In addition, or alternatively, the fluid passage holes 44 may allow the pressure-transmitting fluid to drain out of the interior volume 30 during and/or after processing. While the fluid passage holes 44 are shown as being positioned in a bottom portion of the cylindrical body 22, it will be appreciated that the fluid passage holes 44 may be positioned at any suitable location, and in any suitable quantity.

In addition, or alternatively, auxiliary fluid passage holes 46 may be provided in at least one of the first or second ends 24, 26 of the cylindrical body 22.

Further as shown in FIGURE 1, a plurality of longitudinal indentations 48 may extend along an exterior surface of the sidewall 28 between the first and second ends 24, 26 of the body 22. Such indentations provide the load basket 20 with increased stiffness and/or rigidity. Such indentations may also exist as longitudinal protrusions

The features of load basket 20 described above may be employed in the load baskets described below and shown in FIGURES 2-9. So as to avoid redundancy, the description of such features will not be repeated, but with the understanding that such features may apply to the embodiments of the load baskets shown in FIGURES 2-9.

The present disclosure below provides various embodiments of both load basket and bulk containers designed to address the temperature stratification or layering phenomena that occurs in a typical HPP application. In this regard, load baskets and bulk containers are provided wherein the typical bottom portion of the load basket or container is truncated or filled in so as to eliminate a bottom well section of the basket/container. The cooler, heavier density processing medium or pumpable product tend to collect in the bottom well and thus not mix with the warmer processing medium or pumpable product fluid located above. Also, the product being processed is kept away from the lower portion of the pressure vessel where the colder processing medium tends to collect.

Also, it is desirable that the load basket and/or bulk container retain its orientation in a processing chamber so that the removed or modified lower section of the load basket/container remains in correct orientation. Typically, a processing chamber for receiving the load basket/bulk container is cylindrical in cross section, and does not provide any structure or other means for orienting the load basket or bulk container in any particular rotational position relative to its longitudinal axis.

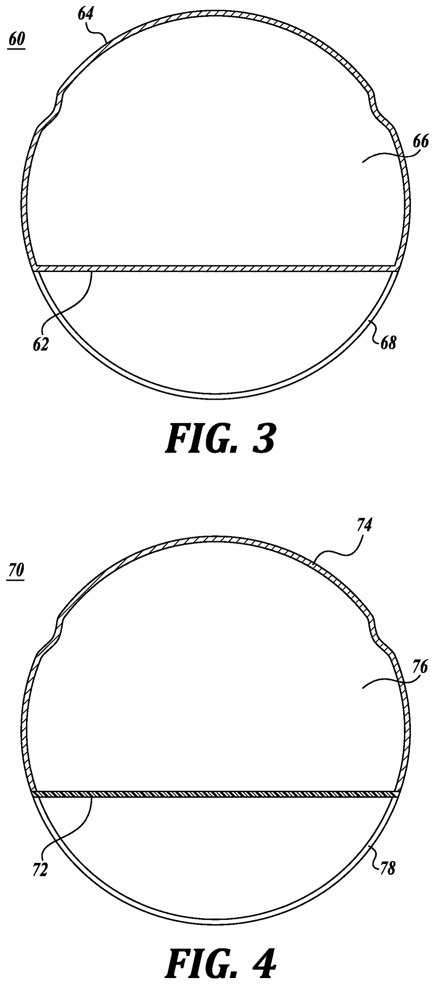

FIGURES 2 and 3 illustrate a load basket 60 that is similar in construction to the load basket 20 of FIGURE 1, with the exception that the bottom portion of the load basket is truncated. A floor 62 extends across the lower portion of the load basket so as to eliminate the majority of the concave interior lower portion of the body portion 64 of the load basket as part of the volume for holding product during HPP processing. As shown in FIGURE 3, the floor 62 may be constructed from the same material as the load basket body portion 64. Also, the floor is shown as substantially flat or horizonal but need not be so. Rather, the floor can be ridged or otherwise contoured. Further, arcuate supports 68 or other supports are used to support the load basket for HPP processing. The supports can be spaced long the length of the body 64. It is to be understood that the other forms of supports can be used to form a closed or an open structure beneath the floor 62.

With the elimination of the concave lower portion of the body portion 64, there is less likelihood that the colder and denser processing fluid circulating through the interior 66 of load basket 60 will be trapped at the bottom of the load basket. Rather, there is more of a likelihood that the processing fluid will circulate through the body portion 64, so as to more thoroughly mix than if the load basket body portion were constructed in a typical circular cross-sectional shape, such as shown in FIGURE 1.

Although not shown, holes may be formed in the floor 62 to enable processing medium to pass through the floor thereby to enhance the mixing of the processing medium within the interior 66 of load basket 60 and within the surrounding high pressure chamber within which the load basket is positioned during HPP.

It can be appreciated that the floor 62 may be located at various distances from the bottom of the body portion 64 that would exist if the body portion were not truncated by the floor 62. Although the floor 62 is illustrated as at an elevation of about 70% of the distance between the nominal bottom of a cylindrical body portion and the diametrical center of the body portion, it will be appreciated that the floor 62 can be at other elevations. As a non-limiting example, the floor may be at an elevation from 20 to 80% of the distance above the bottom of the body portion relative to the center of the nominally cylindrically shaped body portion.

FIGURE 4 is a cross-sectional view of a load basket 70 which is constructed similarly to load basket 60 but with a floor 72 composed of a material that exhibits higher adiabatic properties than the body portion 74. As a consequence, when pressure is applied by the fluid medium in a high-pressure chamber, the floor portion 72 will be heated to a higher temperature than the body portion 74 of the load basket 70. In this regard, the floor 72 can be composed of a material that increases in temperature more than both food and pressure media do. Thus, the processing water that contacts the floor 72 will be heated to a higher temperature than if the floor were composed of the same material as the body portion 74 of the load basket. Such heating of the processing medium adjacent the floor 72 promotes circulation of such processing medium.

Moreover, although not shown, holes can be formed through the thickness of the floor 72. Such holes can function as ejectors, i.e., when the warmer fluid starts to rise inside the vessel the ejectors will pull colder water through the holes, from underneath the floor, and into the interior 76 to get mixed with the warmer water therein.

Also, a support structure 78, which can be similar to support structure 68, can be used to support the load basket for HPP processing.

Various types of materials exhibiting relatively high adiabatic activity can be employed to form the floor 72. For example, such materials might be composed of low-density polyethylene (LDPE), high density polyethylene (HDPE), or ultra high molecular weight polyethylene (UHMWPE).

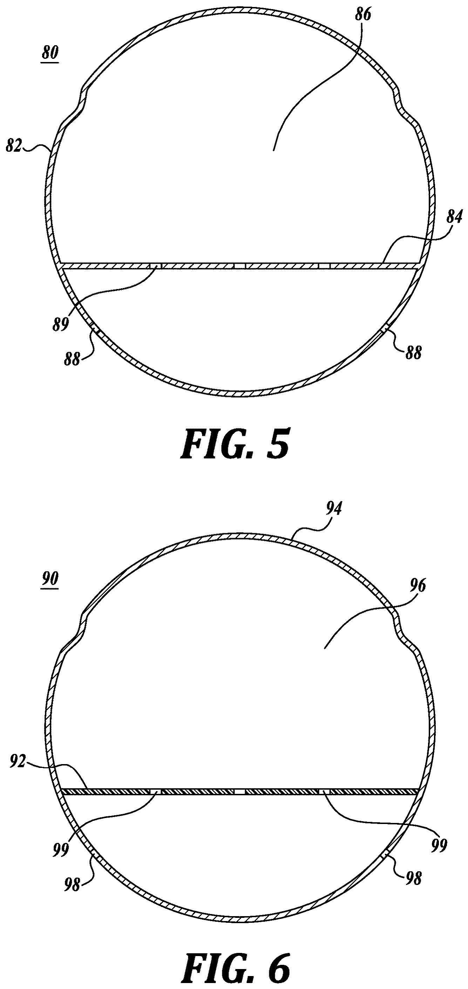

FIGURE 5 illustrates an embodiment of the present disclosure wherein load basket 80 includes a substantially circular cross-sectional shaped body 82 as in FIGURE 1; however, a floor or partition 84 extends across the bottom portion of the interior 86 of the body portion 82 of the load basket so as to effectively truncate the interior volume of the load basket 80 to essentially correspond to the interior volumes 66 and 76 shown in FIGURES 3 and 4. In that the floor or partition 84 serves to truncate the interior volume 86 as in the embodiments discussed above, as such the load basket 80 is considered to have an truncated lower section.

The load basket 80 has the advantage of the rigidity of a load basket in the form of a complete circle or cylinder cross section at least in the middle and ends of the body portion 82, but at the same time preventing the denser, cooler processing medium to collect and remain stationary at the bottom of the load basket interior 86.

The floor 84 may be constructed of the same material as the body portion 82. Also, holes 88 are provided in the load basket below the level of the floor 82, and holes 89 may also be provided in the floor 84 to perform the same function as the holes in floor 72 described above.

FIGURE 6 illustrates a load basket 90 constructed similarly to load basket 80, but with the floor 92 composed of a material with higher adiabatic properties than the body portion 94 remainder of the load basket. As discussed above, with respect to load basket 70, through this construction the floor 92 may be heated to a higher level during pressurization of the load basket 90. As a result, processing medium in contact with the floor 92, though initially perhaps cooler than the processing medium at the upper portion of the load basket, may be sufficiently heated so as to enhance its movement within the interior 96 of the load basket. In that the floor or partition 92 serves to truncate the interior volume 96 as in the embodiments discussed above, as such the load basket 90 is considered to have a truncated lower section.

As with the floor 72, the floor 92 can include through holes 99 formed therein to cause the processing medium heated by the floor to pass upwardly into the interior 96 of the body 94, thereby further facilitating the movement of the processing medium within the interior. Further, as discussed above with respect to load basket 80, openings 98 can be formed in the body portion 94 of the load basket below the floor 92 so that pressurized medium 98 is directed to the floor 92 and then upwardly into the interior 96. Further, as with the floor 72, the floor 94 can be composed of various materials, for example, LDPE, HDPE, and UHMWPE.

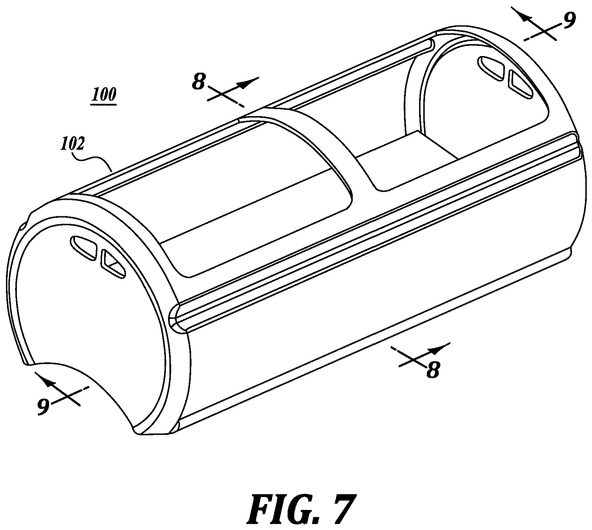

FIGURES 7, 8, and 9 illustrate a further embodiment of the present disclosure wherein the load basket 100 has an upper body portion 102 similar to that shown in FIGURES 2 and 3, but in the place of a transverse floor or partition, the load basket includes a lower carrier portion 104 that serves several functions including as forming the floor surface 106 of the load basket at an elevation that may correspond to the elevation of floors 62, 72, 82, or 92 discussed above as well as supporting the body portion 102. The lower carrier portion 104 serves to truncate the load basket 100 as in the embodiments discussed above. As such the load basket 100 is considered to have a truncated lower section.

Although the carrier portion 102 can be constructed in numerous different configurations and profiles, the configuration shown in FIGURES 7-9 includes a flat upper floor surface 106 and in this case a concave lower bottom surface 108. Moreover, the carrier portion 104 can be composed of substantially thicker cross section than the body portion 102.

As a consequence, by forming the carrier portion 104 from a higher level adiabatic material than the body portion 102, a significant heat source can be provided for heating the processing medium that comes into contact with the surfaces of the carrier portion 104 while also heating the processing medium that flows through the through openings 110 formed in the carrier portion. As explained above, such through openings 110 can function as ejectors wherein the processing medium heated by the carrier portion 104 is ejected into the interior 112 of the load basket.

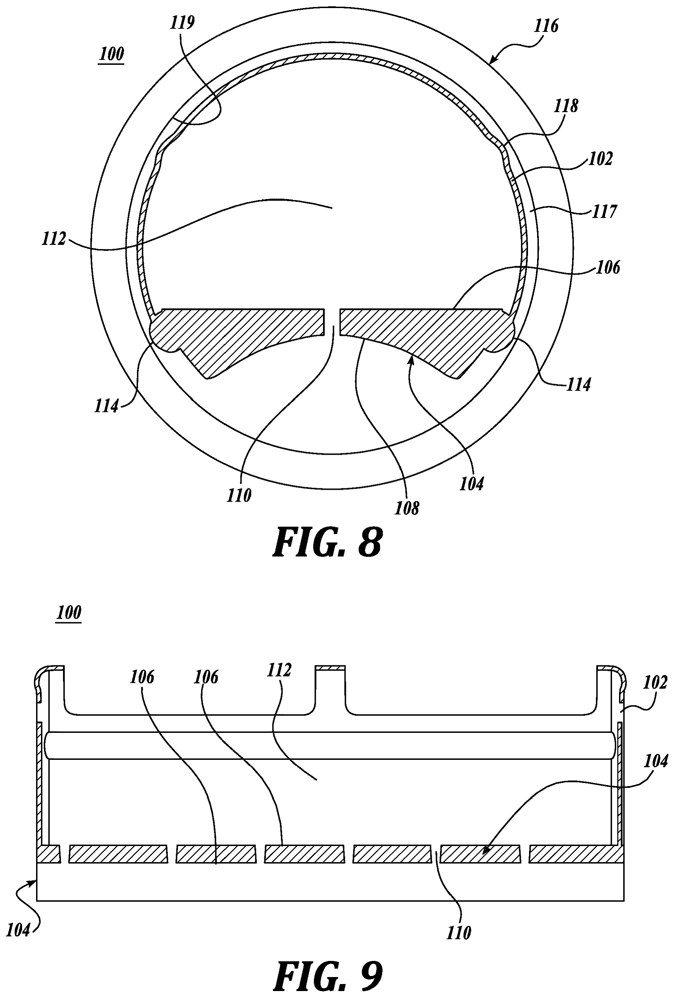

As most clearly shown in FIGURE 8, the carrier portion 104 includes orientation projections 114 extending laterally from each side of the carrier portion and also extending lengthwise of the carrier portion. Such projections serve as low friction slide bearings as the load basket 100 is slid into and out of the high-pressure chamber 116 of a wire wound vessel. The projections 114 also serve as restrictors that restrict the downward movement of processing medium in the clearance gap 117 between the exterior of the load basket body 102 and the interior surface of the high-pressure chamber 116. Otherwise, the cooler, denser processing medium would attempt to flow downwardly through the gap 117 to collect in the bottom portion of the high-pressure chamber.

As also shown in FIGURE 8, projections 118 may in addition extend outwardly from upper portions of the load basket body 102 to help prevent rotation of the load basket 100 once the pressure vessel 116 has been filled with pressure media, e.g., water causing the load basket to rise upwardly within the vessel so that the projections 118 bear against the upper portions of the vessel inside wall. It is to be understood that other means may be employed to prevent rotation of the load basket 100. For example, a rod or other weighted item or structure could be positioned along the length of the carrier portion 104 to serve as a ballast to stabilize the load in the basket when the vessel is filled with pressure media. In this regard, as an alternative, the carrier portion can be greater thickness than the load basket body, and thus serve as a ballast to stably retain the load basket 100 in correct orientation, as shown in FIGURE 8. Of course, other types of ballast can be used.

Although the load basket 100 shown in FIGURES 7-9 is constructed with a carrier portion 114 of a material having higher adiabatic activity than the body portion 102, it is to be understood that carrier portions can be constructed in other configurations wherein the carrier portion is composed of the same material as the body portion 102. In this regard, the wall section of the carrier portion can be substantially thinner than shown in FIGURES 7 and 8. As such, appropriate bracing or reinforcing can be provided so that the thinner constructed carrier portion has sufficient structural integrity to support the filled load basket whether within the high pressure chamber 116 or exterior to the chamber, for example, when being loaded or unloaded.

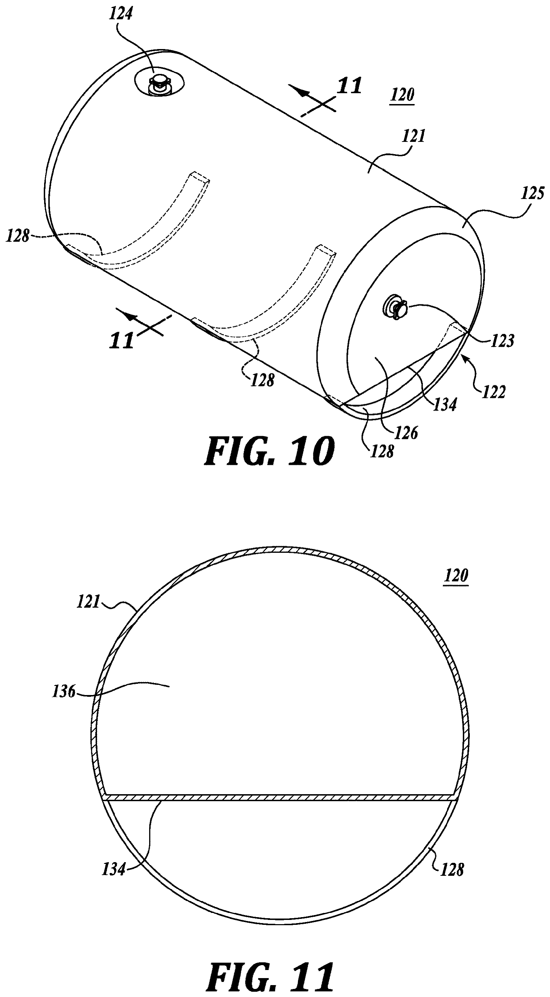

Next, FIGURES 10 and 11 discloses a bulk container 120 for use in an HPP system. The bulk container 120 includes a flexible body portion 121 having a truncated bottom portion or floor 134 as described below. The body portion 121 will first be discussed. In this regard, the body portion includes closed off ends 122, which are depicted as being recessed. Alternatively, one end of the body portion may be concaved inwardly and the other end convexed outwardly. Either configuration enables the containers 120 to be positioned end-to-end in an efficient manner, for example, when placed into an HPP vessel. An inlet closure, for example in the form a valve 123, is located in one or both of the ends 122 of the container 120. Also, one or more outlet closures, for example in the form of valves 124, are located on the body portion 121 of the container for emptying the container, for example, after HPP. Also, a support structure 128, which can be similar to support structures 68 and 78, can be used to support the container 120 for HPP processing.

The body portion 121 is shown as being in the shape of a portion of a cylinder. However, the body 121 can be of other cross-sectional shapes, including as a portion of a pentagon, hexagon, octagon, etc. Also, the body 121 can be of a desired diameter or cross-sectional dimension, as well as of a desired length, so as to provide a desired volume for the bulk container as well as a desired aspect ratio (length v. diameter). Thus, the containers 120 can be of the same diameter, but of different lengths so as to be of various volumes and capacities. In this manner, different beverages or other pumpable products can be processed at the same time, when the different products may be of different quantities.

As noted above, the bulk container 120 can be of various sizes and volumes. For example, the bulk containers can have a capacity as small as of about 20 to 25 liters, to a capacity of at least 200 to 250 liters. In this regard, the smallest capacity bulk containers may have a diameter of about 250 to 300 mm, while the larger containers may have a diameter at least 450 to 475 mm. Of course, the bulk container 120 can be of an even smaller capacity and smaller diameter as well as be of an even larger capacity and a larger diameter.

Referring to FIGURE 10, the ends 122 of the bulk container 120 have a rounded corner 125 that transitions into a concave recess 126. Such rounded corners enable the containers to be placed end to end without damage to the containers, even if the containers are pushed against each other.

It is to be understood that the container end portions 122 can be of a construction and shape other than as shown in FIGURE 10. For example, the end portions 122 may be substantially planar but with a central recess for receiving the inlet valve 123.

The bulk container 120 can be constructed of various materials, which enable the container to maintain its shape while also being sufficiently flexible to adjust to the reduced volume of the product within the container during HPP. Such reduction in volume may be in a range of 0% to up to at least 30%, thereby requiring the volume of the container to be reduced by this same percentage. The material from which the bulk container may be constructed can include, for example, metallic material or polymer material. Such material, as can be appreciated, must be of sufficient flexural strength and sufficient flexural modulus to enable the container to substantially reduce in volume while being rugged enough for reuse over a desired number of HPP cycles. Such HPP cycles may be an indefinite number of cycles. As such the container can be used indefinitely as long as the container cleaned to meet food cleanliness and other standards.

The typical temperature operating range of an HPP operating cycle is from 0°C to 50°C. However, the operating temperature may be higher when HPP is used in conjunction with heat pasteurization wherein the operating temperature may raise to 65°C or perhaps 70°C. The material from which the bulk container 120 is constructed is selected to operate within this temperature range, or perhaps at lower or even higher temperatures.

As mentioned above, the bulk container 120 may be composed of a polymer. As a specific non-limiting example, the polymer may be composed of a thermal plastic, such as polyethylene or nylon. As a further non-limiting example, the polymer may be composed of low-density polyethylene (LDPE), high density polyethylene (HDPE), or ultra-high molecular weight polyethylene (UHMWPE).

As a further non-limiting example, the polymer may have a thickness in the range of from about 4 mm to about 12 mm. The thickness may depend upon several factors, for example, the type of polymer used, the density of the polymer, the diameter of the container, the length of the container, the type of product to be processed, and the pressure level to which the product and container is to be subjected.

The bulk container 120 may be used to process products at high pressures and temperatures than has been the typical operating range for HPP systems. For example, the bulk container may be used operating temperatures of at least 130°C or higher in situations for both elevated temperatures and pressures are used for sterilization. Such operating pressures may be as high as 8,000 bar or even higher. Many thermal plastics are not designed to operate in these elevated temperatures and pressures. However, "high performance" thermoplastics do exist that are capable to successfully operating at such temperatures and pressures, for example polyetheretherketones, polyamideimides, and polyimides. Also, the thermoplastic may be reinforced with fiberglass or carbon fibers to enhance mechanical and/or thermal properties.

Regardless of the material used to construct the bulk container 120, such material must be compliant with applicable safety standards for food or other products being processed at the operating temperatures being used.

As noted above, the inlet closure, for example valve 123, may be positioned in one or both ends 122 of the bulk container 120 in such a manner that the closure, at least when in closed position, is within the outer envelope of the container. This enables the closure to be easily opened and closed, while still protecting the valve from damage, for example, from adjacent containers during HPP.

The outlet closure may be of the same or similar construction to the inlet closure. Such outlet closure is located on the container body 121. The container body 121 at the location of the outlet closure is recessed so that when the outlet closure is in closed position or configuration, the closure remains within the outer perimeter or profile of the container or within the overall length and width of the container body. As such, the outlet closure is protected from being damaged, or causing damage, by undesirable contact with the HPP vessel or other containers or surfaces during filling, during the HPP process, during removal from the HPP vessel, and during other handling of the container, while still being conveniently opened and closed as necessary.

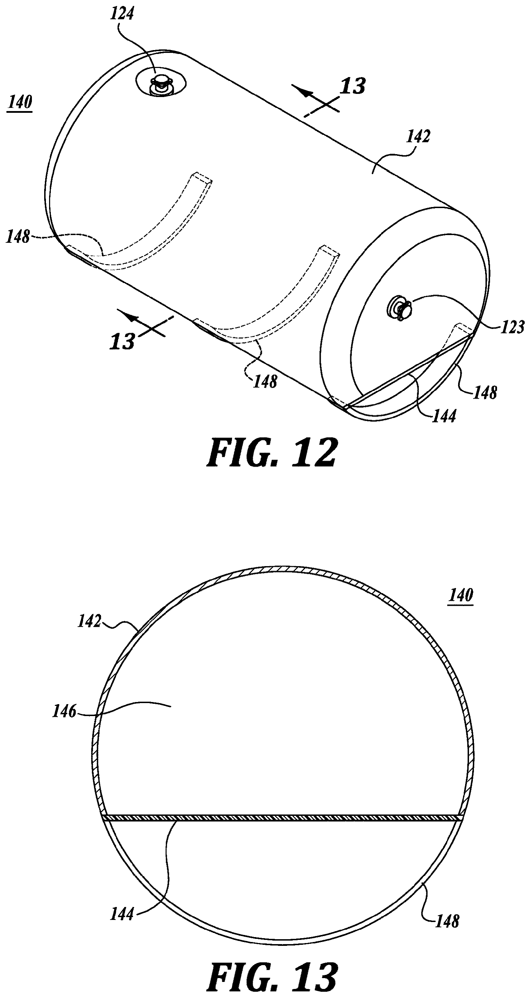

The features of bulk container 120 described above may be employed in the bulk containers described below and shown in FIGURES 12-15. To avoid redundancy, such features will not be repeated, but with the understanding that such features may apply to the embodiments of the bulk containers shown in FIGURES 12-15.

As shown in FIGURES 10 and 11, the bulk container 120 has a body portion 121 generally in the shape of portion of a cylinder, but with its bottom truncated by a floor 134 similar to the manner in which the floor of carrier or load basket 60 is constructed. However, the floor 134 has no through openings or holes therethrough since interior 136 of the bulk container 120 is sealed from the exterior. Nonetheless, the floor 134 functions in the manner of floor 62 as described above. In this respect, the floor 134 constructed from the same material as the body portion121. As such, colder, more dense product to be treated within the bulk container 120 does not tend to collect at the bottom of the interior 136 of the body 121. Rather, the pumpable product within the body 121 tends to circulate and move within the interior 136. This promotes better mixing of the product within the interior 136 due to the presence of the floor 134 above the bottom of the nominal body 121 (i.e., if the body were shaped in a circular cross-sectional profile).

FIGURES 12 and 13 disclose a bulk container 140 that is similar in shape and construction to bulk container 130 except that the floor 144 is composed of an adiabatic material of higher activity than the material from which the body portion 142 of the container is formed. As discussed above, including with respect to load basket 70, constructing the floor 144 in this manner results in the floor being heated to a higher temperature than the body 142 during HPP and perhaps also the temperature of the pumpable product within the interior 146 of the container 140. As such, the pumpable product being treated that comes into contact with the floor 144 will be heated thereby, which in turn facilitates movement and circulation of the pumpable product within the interior 146 of the container. Accordingly, the likelihood of a significant temperature gradient within the interior 146 of the bulk container is countered by not only the presence of a floor 144 that truncates the bottom of the container, but also the higher activity adiabatic material from which the floor is constructed.

Also, a support structure 148, which can be similar to support structures 68, 78 and/or 128, can be used to support the container 140 for HPP processing.

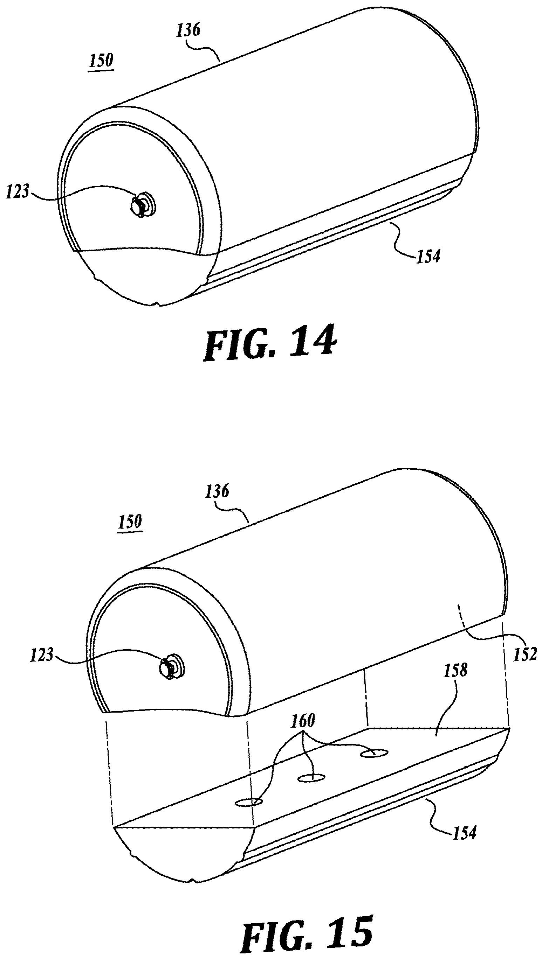

FIGURES 14 and 15 disclose a bulk container 150 that overall has a cylindrical exterior shape, but with an interior volume 152 similar to the interior volumes 136 and 146 of the bulk containers of FIGURES 10-13. In this regard, the bulk container 150 includes a lower carrier portion 154 disposed beneath an upper body portion 156.

As shown in FIGURES 14 and 15, the upper body portion 156 is similar in shape to the body portions 132 and 142 of the bulk carriers shown in FIGURES 10 and 12. However, rather than employing a floor similar to floors 134 and 144, the bottom of the interior volume 152 is closed off by the top surface 158 of carrier portion 154. Thus, the cross-sectional shape of the interior volume 152 within the body portion 156 is similar to that shown in FIGURES 11 and 13. Since the lower carrier portion 154 serves to truncate the interior of bulk container 150 in the manner of the embodiments discussed above, the bulk container 150 is considered to have a truncated lower section.

The carrier portion 154 is of substantially solid construction to form the bottom portion of the bulk container 150 so that the container is of cylindrical construction overall as well as to support the bulk container. Thus, the carrier portion 154 has an outer curved cross-sectional shape corresponding to the circumference of the body portion 156 of the bulk carrier 150. The carrier portion 154 may be attached to the body portion 156 by any convenient means.

Further, the carrier portion 154 may be constructed from a material that may be similar to the construction of the carrier portion 104 shown in FIGURES 8 and 9. As such, the carrier portion 154 will be heated to a higher temperature than the body portion 156 and the contents of the bulk carrier 150 as well as the processing medium used to pressurize the bulk container. As such, and as explained above with respect to carrier portion 104, the higher temperature to which the carrier portion 154 is heated will cause the pumpable product within the interior volume 152 that comes in contact with the top surface 158 to be heated and thereby not remain in the lower portion of the interior volume, but rather to circulate within the interior volume.

A series of blind holes 160 are disposed centrally along the length of the carrier portion. These holes are used to position the body portion 156 relative the carrier portion 154 that includes downwardly extending projections, not shown, to closely engage into the blind holes. Of course, other means can be used to position the body portion 156 relative to the carrier portion 154.

Although not shown, the carrier portion 154 may include projections similar to projections 114 of carrier portion 104 to help restrain the bulk carrier 150 so that the carrier portion remains beneath the body portion 156.

In addition, although rather than being of substantially solid construction, the carrier portion 154 may include cavities or grooves or other features so as to increase the heat transfer area of the carrier portion in a manner analogous to fins in a heat exchanger. As such, the carrier portion would be more efficient in transferring heat from the carrier portion to the work product within the interior volume 152 of the bulk carrier 150.

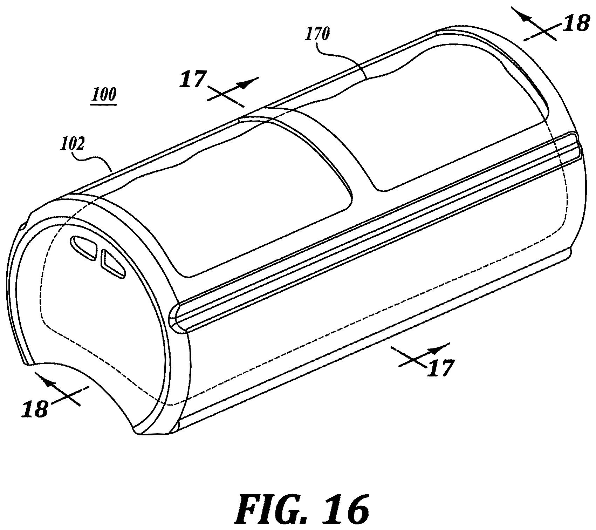

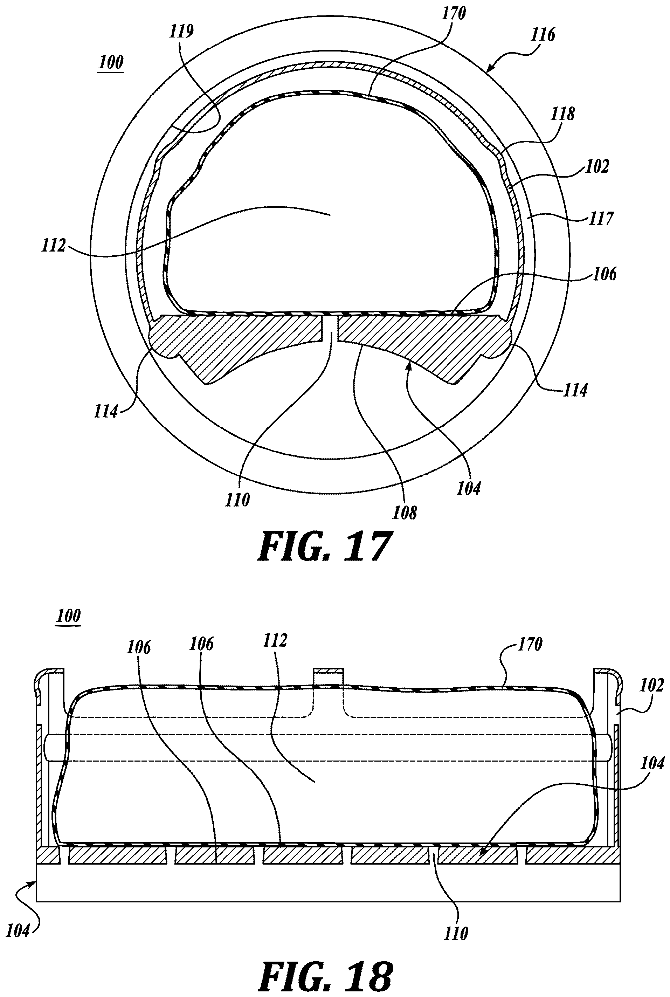

Figures 16, 17, and 18 illustrate an embodiment of the present disclosure wherein a load basket, such as shown in Figures 7, 8, and 9, is employed to hold a large, bulk thin-walled bag or bladder 170 that occupies substantially the entire volume of the interior 112 of the load basket 100. As shown in Figures 16, 17, and 18, the flexible bag 170 compresses or decreases in volume to correspond to the decrease in volume of the bag contents during HPP processing. However, once the pressure of the HPP processing is removed, the bag 170 can resume to its nominal volume of the uncompressed product within the bag.

The bag 170 can be constructed from appropriate commercially available materials, for example rubber or a polymer. Also, appropriate valving maybe utilized in conjunction with the bag 170 for filling and emptying the bag. In addition, the bag may be designed for a single or limited number of uses, or may be constructed for indefinite use. If the bag is designed for more than singular use, it will be necessary to be able to clean and sterilize to bag as required by applicable food handling regulations. Of course, the load basket will also need to be cleaned and sterilized.

The load basket 100 shown in FIGURES 7, 8, 9, 16, 17 and 18 may include a carrier in the manner of, for example, carrier 154 shown in FIGURES 14 and 15, which may be in lieu of, or in addition to carrier 104 to thereby help support the upper body portion 102.

It will be appreciated that the examples of the load baskets and closed containers disclosed above enable pumpable products to be treated by HPP processing whether the products are prepackaged, for instance in bottles or pouches, or in bulk form in a large thin-walled bulk bag or bladder or in a bulk container that retains its shape but is flexible enough to compress with the compression of the product being treated when subjected to high HPP pressure. Further, in such HPP processing the containers are constructed to counteract the vertical thermal stratification or layering that commonly occurs in both the processing medium and the product being processed during HPP processing due to the adiabatic temperature rise in the processing medium and the processed product cause by the high pressures imposed during HPP processing. In this regard the containers of the present disclosure may: (1) restrict or remove usage of the lower portion of the pressure vessel, (2) increase the mixing of the pressure media by use of the injector holes formed in, for example, a floor or partition extending across the container so as to avoid a well in the lower portion of the container in which colder processing medium or pumpable product may collect; and/or (3) are constructed so that the lower portion of the container is composed of material exhibiting higher the adiabatic activity or properties than the upper or main body portion of the container.

It also will be appreciated that the various containers described above can be of different lengths, diameters, weights, volumes, wall thicknesses, materials and other parameters depending on, for example, the application being used for HPP processing as well as available pressure vessel size or capacity. Further, the size or capacity of the container will dictate whether or not the container can be manually handled or if auxiliary handling equipment is needed.

It also can be appreciated that by the above construction of the containers 120, 140, and 150 the contents of the containers can be conveniently and safely stored in appropriate facilities both before and after HPP processing, especially at low temperatures, perhaps close to or at 0°C, so as not to permit microbial growth. This is enhanced by the shape and construction of the containers for ease of handling, as well as by their material composition.

Further, it can be appreciated that the containers, bags, bladders, etc., that hold food or other product to be processed are composed of materials that are suitable therefor, and meet all applicable regulations and standards. Further all load baskets, containers, bags, bladders, etc. and cleanable before and/after use as also required by applicable regulations and standards.

While illustrative embodiments have been illustrated and described, it will be appreciated that various changes can be made therein without departing from the spirit and scope of the invention. For example, in addition to protrusions 114 and supports 68, 78, 128 and 148 discussed above, shims, flanges, rails, bars, runners, slides, circumferentially extending hoop or ring sections or members, and other or similar structures can be used to extend or protrude beneath the container, whether a load basket or a closed container, for supporting the container during the HPP process.

The invention may be summarized as follows:
Item 1: A container or load basket constructed for addressing thermal stratification of the product in the container or load basket and addressing thermal stratification of the processing medium during high pressure processing with a pressurized processing medium, the container comprising:
   an upper section forming a volume for receiving the product to be processed; and
   a truncated lower section forming the bottom of the container.
Item 2. The container or load basket of Item 1, wherein the bottom of the container is defined by a floor or partition extending across the container.
Item 3. The container or load basket of Item 2, wherein the floor extends across the bottom of the container.
Item 4. The container or load basket of Item 2, wherein the floor or partition extends across the lower portion of the container.
Item 5. The container or load basket of any one of Items 2-4, wherein the floor or partition is constructed from a group consisting of: the same material from which the upper section of the container is formed; and, a material that is more adiabatically active than the material from which the upper section of the container is formed.
Item 6. The container or load basket according to any one of Items 2-5, wherein the floor or partition includes passageways extending therethrough for passage of the high pressure processing medium.
Item 7. The container or load basket according to Item 1, wherein the truncated lower section of the container is formed from a material selected from the group consisting of: the material from which the upper section is formed; and, a material that is more adiabatically active than the material from which the upper section is formed.
Item 8. The load basket according to any one of Items 1-7, further comprising through openings formed in the load basket through which the processing medium may pass.
Item 9. The container or load basket of Items 1-8, wherein the container further comprising first orientation projections extending lengthwise of the container for serving as slides when the container is moved lengthwise.
Item 10. The container or load basket of any one of Items 1-9, further comprising second orientation projection or orientation indentations extending lengthwise of the upper section of the container or along the container lower section above the first orientation projections.
Item 11. The container or load basket of any one of Items 5 or 7, wherein the material that is more adiabatically active is composed of a material selected from LDPE, HDPE, and UHMWPE.
Item 12. The container or load basket according to Item 1, wherein the truncated lower section comprises a carrier portion disposed beneath the container upper section, said carrier portion defining the floor of the container and supporting the weight of the container.
Item 13. The container or load basket according to Item 12, wherein the carrier portion defining first orientation projections extending from and along the length of the carrier portion to assist in retaining the carrier portion beneath the upper section during high pressure processing.
Item 14. The container or load basket according to Item 12 or 13, wherein the carrier portion comprising additional orientation projections extending from and lengthwise of the carrier portion at a position above the first orientation projections of the carrier portion.
Item 15. The container or load basket according to any one of Items 12-14, wherein passageways extend through the carrier portion to enable passage of the high pressure medium therethrough.
Item 16. The container or load basket according to any one of Items 12-15, wherein the floor of the carrier portion is contoured to increase the surface area of the floor.
Item 17. The container or load basket according to any one of Items 12-16, wherein the carrier portion is composed of a material selected from the group including the material from which the upper section of the container is formed and a material that is more highly adiabatically active than the material from which the upper section of the container is formed.
Item 18. The container or load basket according to Item 17, wherein the material that is more adiabatically active is composed of a material selected from LDPE, HDPE and UHMWPE.
Item 19. The container or load basket according to any of the above Items, wherein the openings are provided through which product is receivable into the container and removable from the container as well as providing access for the processing medium to enter and exit the container.
Item 20. The container or load basket according to any of the above Items, further comprising a flexible, thin-walled bulk bag or bladder disposed within the container and occupying up to substantially the entire volume of the container, the bag or bladder configured to receive and hold pumpable products during high pressure processing and disgorge the pumpable product after high pressure processing.
Item 21. The container according to any one of Items 1-5, 7, 9-14, and 16-18, wherein the container is a closed flexible configuration to hold product during high pressure processing and to reduce in volume corresponding to the reduction in volume of the product occurring during high pressure processing.
Item 22. The container or load basket according to any one of the above Items, further comprising a support selected from the group consisting of a shim, flange, a rail, a bar, a runner, a slide, a transverse ring section, a transverse hoop section protruding beneath the container or load basket for supporting the container or load basket during the HPP process.

## Claims

1. A generally cylindrically shaped container (70; 80; 90; 100) for receiving and supporting products for high pressure processing (HPP) with a pressurized liquid processing medium within a HPP chamber, the container comprising:
a plurality of fluid passage holes (44, 88, 89, 110) for passage of the liquid processing medium;
an upper body portion (102) forming a volume for receiving the products to be processed; and
a truncated lower carrier portion (62, 72, 92, 104) forming a floor surface (106) and wherein the truncated lower carrier portion (62, 72, 92, 104) is formed from a higher level adiabatic material than the upper body portion (102).

2. The container of Claim 1, wherein the bottom of the container (70; 80; 90; 100) is defined by a floor or partition extending across the container.

3. The container according to Claim 2, wherein the floor or partition includes passageways extending therethrough for passage of the processing medium.

4. The container of any one of Claims 1-3, wherein the container (70; 80; 90; 100) further comprising first orientation projections (114) extending lengthwise of the container for serving as slides when the container is moved lengthwise.

5. The container of any one of Claims 1-4, further comprising second orientation projections (118) or orientation indentations extending lengthwise of the upper section of the container (80; 80; 90; 100) or along the container lower section above the first orientation projections (114).

6. The container according to Claim 1, wherein the carrier portion (104) defining first orientation projections (114) extending from and along the length of the carrier portion to assist in retaining the carrier portion beneath the upper section (102) during HPP.

7. The container according to Claim 1, wherein the carrier portion (104) comprising additional orientation projections (118) extending from and lengthwise of the carrier portion (104) at a position above the first orientation projections (114) of the carrier portion.

8. The container according to any one of Claims 1-7, wherein the floor of the carrier portion is contoured to increase the surface area of the floor.

9. The container according to any of the above claims, wherein the openings are provided in the container (70; 80; 90; 100) through which product is receivable into the container (70; 80; 90; 100) and removable from the container as well as providing access for the processing medium to enter and exit the container.

10. The container according to any of the above claims, further comprising a flexible, thin-walled bulk bag (170) or bladder disposed within the container (70; 80; 90; 100) and occupying up to substantially the entire volume of the container, the bag or bladder configured to receive and hold pumpable products during HPP and disgorge the pumpable product after HPP.

11. The container according to any one of Claims 1-2, and 4-6, wherein the container (70; 80; 90; 100) is a closed flexible configuration to hold product during HPP and to reduce in volume corresponding to the reduction in volume of the product occurring during HPP.

12. The container according to any one of the above claims, further comprising a support (128, 148) selected from the group consisting of a shim, flange, a rail, a bar, a runner, a slide, a transverse ring section, a transverse hoop section protruding beneath the container (70; 80; 90; 100) or load basket for supporting the container or load basket during the HPP.
